# EUROPEAN PATENT APPLICATION

(11) **EP 1 457 213 A1**
(43) Date of publication of application: **15.09.2004**
(21) Application number: 04251323.4
(22) Date of filing: 08.03.2004
(51) Int. Cl.: A61K 47/48

(54) **Anthelmintic resinates and a method for their preparation**

(30) Priority: 10.03.2003 US 453254 P
(71) Applicant: ROHM AND HAAS COMPANY, Philadelphia, Pennsylvania 19106-2399 (US)
(72) Inventor: Hughes, Lyn, Harleysville Pennsylvania 19438 (US); Ziarno, Witold Andrew, Horsham Pennsylvania 19044 (US)
(74) Representative: Kent, Venetia Katherine

(57) **Abstract**

The invention provides a pharmaceutical composition that includes a non-ionizable anthlemintic drug or derivative thereof loaded onto an anion exchange resin or a cation exchange resin. The non-ionizable anthlemintic is praziquantel, a praziquantel derivative, epsiprantel, or an epsiprantel derivative. The anthlemintic can also include Droncit, a derivative of Droncit, a precursor of Droncit, Drontal, a precursor of Drontal, a derivative of Drontal, Drontal Plus, a derivative of Drontal Plus, a precursor of Drontal Plus, a formulation comprising Praziquantel and Pyrantel Pamoate, and a formulation comprising Praziquantel, Pyrantel Pamoate and/or Febantel. In another variant, the invention includes a pharmaceutical composition including a basic anthlemintic drug loaded onto an anion exchange resin, or an acidic anthlemintic drug loaded onto a cation exchange resin, and a process for manufacturing the pharmaceutical composition.

## Description

### BACKGROUND OF THE INVENTION

The present invention generally relates to compositions containing pharmacologically active anthelmintics loaded onto ion exchange resins. In one particular variant, the patent application relates to taste masking processes for hexahydropyrazine derivatives, praziquantel and epsiprantel, and to a taste masked version of praziquantel and a taste masked version of epsiprantel.

Using a complex formed between a polymeric material and an anthelmintic active substance can be beneficial. Such benefits can include changes in the release rate of drugs, taste masking of bitter drugs, control of the site of administration of drugs, control of the release of flavor substances, and stabilization of unstable substances.

The preparation of an active substance/ion exchange resin complex is called loading. The ion exchange resins complexed with the active substance are called resinates.

Basic drugs can be loaded onto cation exchange resins because basic molecules form cations, and acidic drugs can be loaded onto anion exchange resins because acidic molecules form anions. Prior to the present invention the prevailing belief in the art was that non-ionizable molecules cannot be loaded onto ion exchange resins because they cannot form either anions or cations. Further, it was believed in the art that basic molecules cannot be loaded onto anion exchange resins because the molecules do not form anions, and that acidic drugs cannot be loaded onto cation exchange resins because the molecules do not form cations. The inability to load non-ionizable drugs onto ion exchange resins has been a significant limitation to their use because approximately 30% of all active substances used in the pharmaceutical industry are non-ionizable.

An aspect of using polymers for the in vivo delivery of active substances is that the anthelmintic active substance be released from the polymer at some point after administration.

Applicants have surprisingly discovered how to load non-ionizable molecules onto ion exchange resins in such a way that the molecule is efficiently released *in vivo.* Further, Applicants have discovered how to load acidic drugs onto cation exchange resins and how to load basic drugs onto anion exchange resins in such a way that the molecule is efficiently released in vivo. Resinates so formed can have properties that make them useful in the delivery of active substances.

Specific problems in the art are encountered, by way of example, with commercially valuable pharmaceutically active substances, e.g. praziquantel. Praziquantel and other hexahydropyrazine derivatives are known from U.S. Pat. No. 4,001,411, EP-A 13498, EP-A 185 012. The structural formulae and the individual compounds which are mentioned therein are expressly incorporated herein by reference. Of particular commercial significance is: praziquantel: (2-cyclohexylcarbonyl)-1,3,3,6,7-11b-hexahydro-4H-pyrazino[2,1-a]-isoquino lin-4-one; and, epsiprantel: 2-(cyclohexylcarbonyl)-2,3,6,7,8,12b-hexahydro-pyrazino[2,1-a]benzazepin-3 (1H)-one.

Dipyllidium caninum is a common tapeworm of dogs and cats, and is usually targeted by Praziquantel. Praziquantel is also effective against less common types of tapeworms such as the Taenia species and the Mesocestoides species. Praziquantel is also effective against flukes. A single treatment of Praziquantel should clear a Dipyllidium caninum infection. However, a second treatment is recommended if immediate reinfection is likely. Immediate reinfections occur generally if a heavy uncontrolled flea problem is present in the animal's environment. Praziquantel's anthelmintic spectrum of activity for dogs is as follows: Dipyllidium caninum, Taenia pisiformis, Echinococcus multilocularis and E. granulosus. For cats, Praziquantel's anthelmintic spectrum of activity is as follows: Dipyllidium caninum, and Taenia taeniaeformis.

Praziquantel acts by damaging the parasite's skin such that the parasite disintegrates, and is removed by the host's immune system. Praziquantel modulates the parasite's cell membrane permeability (calcium dependent), and leads to a disintegration of the tapeworm's tegument. In particular, it causes the tapeworm to lose its resistance to digestion by host, and causes instantaneous tetanic contraction of parasite muscles and rapid vacuolization of the tapeworms syncytial tegument

Praziquantel is generally injected. However, injectable Praziquantel has the drawback of stinging so strongly at the site of administration that it is not unusual for an animal to scratch at the site or howl immediately post injection. The oral form of Praziquantel has further drawbacks. An exemplary oral form is described in United States Patent 6,503,536 to Kalbe , et al. issued January 7, 2003 and entitled: "Granulates of hexahydropyrazine derivatives which can be administered orally." The oral form of Praziquantel is bitter tasting and at least one out of twenty animals taking it experience nausea. It has further been reported that approximately one cat in ten will experience weakness, salivation, or nausea after Praziquantel injection. As such, many animals are unnecessarily stressed during treatment for tapeworm with injectable Praziquantel. Moreover, the oral form is very bitter tasting when added to a food source that animals do not desire to eat it.

This becomes a particular problem when wild animal populations, such as foxes, are the targets for treatment with Praziquantel. Wild animal populations are targets for treatment since it is believed that tapeworm migrates from wild animal populations where is it endemic, e.g. wild fox populations, to domestic animals, e.g. dogs, cats, and horses. Capturing and injecting wild animals becomes cost prohibitive, and is not a viable option. Moreover, wild animals will not eat a food source intentionally laced with Praziquantel due to the bitter taste of the Praziquantel.

It is an object of the invention to solve the problems in the art.

### SUMMARY OF THE INVENTION

The present invention provides an pharmaceutical composition that includes a non-ionizable anthelmintic loaded onto an anion exchange resin or a cation exchange resin.

In one variant of the invention, the non-ionizable drug is praziquantel or derivative thereof.

In another variant of the invention, the non-ionizable drug is epsiprantel or a derivative thereof.

In yet a further aspect, the anthlemintic is selected from the group consisting of Droncit, a derivative of Droncit, a precursor of Droncit, Drontal, a precursor of Drontal, a derivative of Drontal, Drontal Plus, a derivative of Drontal Plus, a precursor of Drontal Plus, a formulation comprising Praziquantel and Pyrantel Pamoate, and a formulation comprising Praziquantel, Pyrantel Pamoate and/or Febantel.

It is yet a further object of the invention to provide a composition that includes a therapeutically effective dosage to treat a mammal.

In yet a further embodiment, the composition further includes a formulation readily consumable by a mammal in a food product. The therapeutically effective dosage is a dosage to treat a mammal selected from the group consisting of a domestic mammal, a wild mammal, a cat, a dog, a horse and a fox. In yet another variant, the therapeutically effective dosage is in the range of 3 to 100 mg/kg. In yet a further aspect, the therapeutically effective dosage provides for an anthelmintic spectrum of activity against one or more of *Dipyllidium caninum, Taenia pisiformis, Echinococcus multilocularis, E. granulosus,* and *Taenia taeniaeformis, Toxocara, Ancylostoma, Uncinaria, Toxascaris,* and *Trichuris.* In yet another aspect the pharmaceutical composition is given periodically.

In yet a further aspect the invention relates to an anthelmintic pharmaceutical composition comprising a non-ionizable drug or derivative thereof loaded onto an anion exchange resin or a cation exchange resin. The non-ionizable anthelmintic drug is a therapeutic composition in a non-ionized form.

In yet another variant, the invention provides a pharmaceutical composition comprising a basic anthelmintic drug loaded onto an anion exchange resin.

In yet another variant, the invention provides a pharmaceutical composition comprising an acidic anthelmintic drug loaded onto a cation exchange resin.

In yet another aspect, the invention provides a process for manufacturing a pharmaceutical composition, comprising, loading onto a resin a non-ionized form of an anthelmintic drug or derivative thereof.

In yet another variant, the invention provides a process for manufacturing a pharmaceutical composition that includes loading onto an anion exchange resin in a non-ionized form a basic anthelmintic drug or derivative thereof in a non-ionized from.

In yet a further aspect, the invention provides a process for manufacturing a pharmaceutical composition that includes loading onto a cation exchange resin in a non-ionized form an acidic anthelmintic drug or derivative thereof in a non-ionized from.

In yet a further variant of the invention, a composition is provided that includes an ion exchange resin and an active anthelmintic substance wherein the ion exchange resin is a cation exchange resin and the active anthelmintic substance is either acidic or non-ionizable.

It is yet another object of the invention the provide a composition comprising an ion exchange resin and an active anthelmintic substance wherein the ion exchange resin is an anion exchange resin and the active anthelmintic substance is either basic or non-ionizable.

These and other objects of the invention are described here and in other portions of the specification.

### DETAILED DESCRIPTION OF THE INVENTION

The present invention provides an pharmaceutical composition that includes a non-ionizable anthelmintic loaded onto an anion exchange resin or a cation exchange resin. The non-ionizable drug is praziquantel or derivative thereof. In another variant of the invention, the non-ionizable drug is epsiprantel or a derivative thereof. The anthlemintic is selected from the group consisting of Droncit, a derivative of Droncit, a precursor of Droncit, Drontal, a precursor of Drontal, a derivative of Drontal, Drontal Plus, a derivative of Drontal Plus, a precursor of Drontal Plus, a formulation comprising Praziquantel and Pyrantel Pamoate, and a formulation comprising Praziquantel, Pyrantel Pamoate and/or Febantel. Praziquantel is generally used to treat parasitic infestations commonly known as "Cestodes" (tapeworms) and trematodes. Praziquantel is an acylated quinoline-pyrazine. Praziquantel is sold under the tradenames: Droncit®, Drontal®, and Drontal Plus@. Drontal® contains Praziquantel and Pyrantel Pamoate. Drontal Plus@ contains Praziquantel, Pyrantel Pamoate and Febantel, and commercially available from Bayer. It is also commercially available from Merial under the tradename RM ®Parasiticide-10. Its index name is 4H-Pyrazino[2,1-a]isoquinolin-4-one, 2-(cyclohexylcarbonyl)-1,2,3,6,7,11b-hexahydro- (9CI). It is also has other commercial forms that are known as: Azinox; Biltricide; Distocide; Droncit; Embay 8440; Prazinon; Pyquiton; Cesol, and Cysticide. All of these forms and derivatives thereof are useful in the current invention.

It is yet a further object of the invention to provide a composition that includes a therapeutically effective dosage to treat a mammal. A convenient way to administer the formulation is in tablet form or other formulation readily consumable by a mammal in a food product. The therapeutically effective dosage is a dosage to treat a mammal selected from the group consisting of a domestic mammal, a wild mammal, a cat, a dog, a horse and a fox. Other therapeutically effective dosages are dosages to treat fish that include productive and breeding fish, fish for aquariums and ornamental fish of all ages which live in fresh water, salt water and brackish water. The productive and breeding fish include, for example, carp, eel, trout, white fish, salmon, bream, roach, rudd, chub, sole, plaice, halibut, Japanese yellowtail (Seriola quinqueradiata), Japanese eel (Anquilla japonica), red seabream (Pagurus major), seabass (Dicentrarchus labrax), grey mullet (Mugilus cephalus), pompano, gilthread seabream (Sparus auratus), tilapia ssp., chichlid species, such as, for example, plagioscion, channel catfish.

In yet a further variant of the invention therapeutically effective dosages are determined for productive and breeding animals including, for example, cattle, horses, sheep, pigs, goats, camels, water buffalo, donkeys, rabbits, fallow deer, reindeer, fur-bearing animals, such as, for example, mink, chinchilla, racoon, birds, such as, for example, hens, geese, turkeys, ducks, and ostriches.

In yet another variant, the therapeutically effective dosage is in the range of 3 to 100 mg/kg. In yet another variant, a 10 mg/kg dosage used formulated. It is appreciated that one can use methods to provide dosages that provide for an anthelmintic spectrum of activity against one or more of *Dipyllidium caninum, Taenia pisiformis*, *Echinococcus multilocularis*, *E*. *granulosus,* and *Taenia taeniaeformis*, *Toxocara, Ancylostoma, Uncinaria*, *Toxascaris,* and *Trichuris.* Generally a single administration should be adequate to rid the mammal of these organisms. However, the pharmaceutical composition is given periodically where and when needed.

The formulations and therapeutically effective dosages according to the invention are suitable for controlling pathogenic endoparasites. They are active against all or individual stages of development of the endoparasites and also against resistant and normally sensitive species. The pathogenic endoparasites include cestodes, trematodes, nematodes, Acantocephalae, in particular: From the order of the Pseudophyllidea, for example Diphyllobothrium spp., Spirometra spp., Schistocephalus spp.; From the order of the Cyclophyllidea, for example Mesocestoides spp., Anoplocephala spp., Paranoplocephala spp., Moniezia spp., Taenia spp., Echinococcus spp., Hydatigera spp., Diorchis spp., Dipyllidium spp., Joyeuxiella spp., Spyrometra spp.; from the subclass of the Digenea, for example Schistosoma spp., Fasciola spp., Dicrocoelium spp., Opisthorchis spp.; from the order of the Enoplida, for example Trichuris spp., Capillaria spp., Trichinella spp.; from the order of the Rhabditia, for example Micronema spp., Strongyloides spp.; from the order of the Strongylida, for example Stronylus spp., Triodontophorus spp., Oesophagodontus spp., Trichonema spp., Gyalocephalus spp., Poteriostomum spp., Cyclicocyclus spp., Stephanurus spp., Ancyclostoma spp., Uncinaria spp., Cyathostomum spp., Metastrongylus spp., Dictyocaulus spp., Muellerius spp., Protostrongylus spp., Elaphostrongylus spp., Parelaphostrongylus spp., Crenosoma spp., Paracrenosoma spp., Filaroides spp., Parafilaroides spp., Marshallagia spp., Hyostrongylus spp., Ollulanus spp., Craterostomum spp., Cyclicodontophorus spp., Hyalocephalus spp., Cylindropharynx spp., Caballonema spp., Elaeophorus spp., Dirofilaria spp., Onchocerca spp., Setaria spp.; from the order of the Oxyurida, for example Oxyuris spp., Enterobius spp.; from the order of the Ascaridia, for example Ascaris spp., Toxascaris spp., Toxocara spp., Parascaris spp., Probstmangria spp.; from the order of the Spirurida, for example Thelazia spp., Habronema spp., Draschia spp., Dracunculus spp.

In yet a further variant, the anthelmintic pharmaceutical composition includes a non-ionizable drug or derivative thereof loaded onto an anion exchange resin or a cation exchange resin. The non-ionizable anthelmintic drug is a therapeutic composition in a non-ionized form. In yet another variant, the invention provides a pharmaceutical composition comprising a basic anthelmintic drug loaded onto an anion exchange resin. In yet a further variant, the invention provides a pharmaceutical composition comprising an acidic anthelmintic drug loaded onto a cation exchange resin.

There are several processes that can be used to manufacture a pharmaceutical composition of the present invention. For example, it can be made by loading onto a resin a non-ionized form of an anthelmintic drug or derivative thereof; loading onto an anion exchange resin in a non-ionized form a basic anthelmintic drug or derivative thereof in a non-ionized from; or, loading onto a cation exchange resin in a non-ionized form an acidic anthelmintic drug or derivative thereof in a non-ionized from. The terms "loaded" and "loading" means the preparation of a resinate. The amount of loading means the amount of active substance incorporated into the resin to form a resinate. The term "resinate," as used herein, means an active substance/ion exchange resin complex.

Ion exchange resins useful in the present invention are manufactured in different forms. By way of example, these forms can include spherical and non-spherical particles with size in the range of 0.001mm to 2mm. The non-spherical particles are frequently manufactured by grinding of the spherical particles. Products made in this way typically have particle size in the range 0.001mm to 0.2mm. The spherical particles are frequently known in the art as 'Whole Bead.' The non-spherical particles are frequently known in the art as 'Powders.'

The term "water retention capacity" as used herein is used to describe the maximum amount of water that an ion exchange resin can retain within the polymer phase and in any pores. (ASTM D2187: Standard Test Methods for Physical and Chemical Properties of Particulate Ion Exchange Resin. Test Method B: Water Retention Capacity).

In yet a further variant of the invention, a composition is provided that includes an ion exchange resin and an active anthelmintic substance wherein the ion exchange resin is a cation exchange resin and the active anthelmintic substance is either acidic or non-ionizable.

It is yet another object of the invention, a composition is provided that includes an ion exchange resin and an active anthelmintic substance wherein the ion exchange resin is an anion exchange resin and the active anthelmintic substance is either basic or non-ionizable.

The term anion exchange resin as used herein, means an ion exchange resin in which the functional group is basic, such as, by way of example, a primary amine, a secondary amine, a tertiary amine, and a quaternary amine. Further, ion exchange resins are characterized by their capacity to exchange ions. This is expressed as the "Ion Exchange Capacity." For cation exchange resins the term used is "Cation Exchange Capacity," and for anion exchange resins the term used is "Anion Exchange Capacity." The ion exchange capacity is measured as the number equivalents of an ion that can be exchanged and can be expressed with reference to the mass of the polymer ( herein abbreviated to "Weight Capacity") or its volume (often abbreviated to "Volume Capacity"). A frequently used unit for weight capacity is "milliequivalents of exchange capacity per gram of dry polymer." This is commonly abbreviated to "meq/g."

The anthelmintic resinate is prepared by mixing a solution of an active anthelmintic substance with the selected ion exchange resin in a suitable solvent. The ion exchange resin is used in its non-ionized form. An example of the functional group of a weakly acidic cation exchange resin in its non-ionized form is -CO₂H. An example of the functional group of a weakly basic cation exchange resin in its non-ionized form is -N(CH₃)₂. In the case of the weakly acid cation exchange resin, a small amount of a strong acid such as hydrochloric acid can be added to ensure suppression of ionization. The mixture is then mixed for a suitable length of time, and the liquid is removed by filtration. Excess liquid, which may still contain some of the active substance dissolved therein can be removed by washing with a less hydrophobic solvent. For example if the loading is done using water containing 25% by weight ethanol, then the washing can be done using water.

For an acidic active anthelmintic substance, the resin used is a cation exchange resin. For a basic active anthelmintic substance, the resin used is an anion exchange resin. For a non-ionizable active substance both cation and anion exchange resins can be used.

The combination of ion exchange resin and solvent is chosen by methods known by those skilled in the art. Solvents of various hydrophobicity are tested and the solvent is selected that gives the desired loading. For example, a series of test can be done using various mixtures of water and ethanol, such as 0%, 10% 25%, 50% and 100% ethanol by weight.

The ratio of ion exchange resin to solvent is selected to give the desired amount of loading. It is not necessary that the amount of solvent be sufficient to dissolve all of the active substance.

The resinates have been found to release the active anthelmintic substance when exposed to solutions containing ions that cause the resin to change to its ionized form, for example resinates of weakly acidic cation exchange resins in the presence of simulated intestinal fluid of composition as defined by the United States Pharmacopeia. Aqueous fluids that do not cause the resin to ionize do not result in the efficient release of the active substance. For example, resinates of weakly acidic cation exchange resins in the presence of simulated gastric fluid of composition as defined by the United States Pharmacopeia.

In one variant of the invention, the loading of the active substance occurs by adsorption or absorption because the resin in its non-ionized form is significantly hydrophobic. By careful selection of the solvent hydrophobicity it is possible to create conditions where the equilibrium between being in solution or being adsorbed or adsorbed onto the polymer matrix is strongly in favor of the polymer matrix. However, when the resinate is exposed to ionic solutions such as gastrointestinal fluids, the resin changes into an ionized state. This ionized state is much more hydrophilic than the un-ionized state so that the equilibrium is shifted toward the solution, and so the active substance is released back into solution. The applicants have used the term 'reversible hydrophobicity' to describe this novel concept.

While this is one example of reversible hydrophobicity, reversible hydrophobicity can be accomplished by other methods. For example, the presence of a third component that makes the ion exchange resin hydrophobic could be released *in vivo,* rendering the ion exchange resin hydrophilic and thence releasing the active substance. Examples of these third components include: anionic and cationic surfactants

Ion exchange resins useful in the practice of the present invention include, but are not limited to, weakly basic anion exchange resins and weakly acidic cation exchange resins. Preferably, said resins are suitable for human and animal ingestion.

Anion exchange resins include, but are not limited to, styrenic weakly basic anion exchange resins with a primary, secondary, or tertiary amine functionality having a weight capacity of 0.1 to 8.5 meq/g, and acrylic or methacrylic weakly basic anion exchange resins with a primary, secondary, or tertiary amine functionality having a weight capacity of 0.1 to 12 meq/g, and allylic and vinylic weakly basic anion exchange resins with a primary, secondary, or tertiary amine functionality having a weight capacity of 0.1 to 24 meq/g.

Preferred anion exchange resins include, but are not limited to, styrenic weakly basic anion exchange resins with tertiary amine functionality having a weight capacity of 0.1 to 8.5 meq/g, and acrylic or methacrylic weakly basic anion exchange resins with tertiary amine functionality having a weight capacity of 0.1 to 12 meq/g, and allylic weakly basic anion exchange resins with a primary, secondary, or tertiary amine functionality having a weight capacity of 0.1 to 24 meq/g.

Cation exchange resins include, but are not limited to, styrenic strongly acidic cation exchange resins with sulfonic or phosphonic acid functionalities having a weight capacity of 0.1 to 8 meq/g; and styrenic weakly acidic cation exchange resins with carboxylic or phenolic acid functionalities having a weight capacity of 0.1 to 8.5 meq/g; and acrylic or methacrylic weakly acidic cation exchange resins with a carboxylic or phenolic acid functionality with a weight capacity of 0.1 to 14 meq/g.

Preferred cation exchange resins include, but are not limited to, acrylic or methacrylic weakly acidic cation exchange resins with a carboxylic acid functionality with a weight capacity of 0.1 to 14 meq/g.

Ion exchange resins useful in this invention have a moisture content between 0% and the water retention capacity of the resin. Moreover, ion exchange resins useful in this invention are in powder or whole bead form. Ion exchange resins useful in this invention are in their non-ionized form during the loading procedure.

Active anthelmintic substances useful in the practice of this invention must be non-ionizable or capable of existing in a non-ionized state. By way of example, active anthelmintic substances useful in the practice of the invention include: praziquantel and epsiprantel

Solvents useful in the practice of the present invention include, but are not limited to, water, methanol, ethanol, isopropanol, n-propanol, acetone, dimethylformamide, tetrahydrofuran, dimethyl sulfoxide, dimethyl ether, acetic acid, and mixtures thereof. By way of example, the preferred solvents are water, methanol, ethanol, isopropanol, n-propanol, and mixtures thereof. The most preferred solvents are mixtures of water and ethanol, and water with isopropanol. The active ingredients are used in the pharmaceutical compositions of the present invention at levels of 2 - 60 weight %, preferably, 5 - 40 weight % percent, and most preferably, 5 - 30 weight %.

The following non-limiting examples illustrate the practice of the present invention.

### EXAMPLE 1

Praziquantel loaded onto a cation exchange resin. - In this example, the resin used was a weakly acid, methacrylic, cation exchange resin, with a an exchange capacity of approximately 10.6meq/g. Water (5.63kg) and 95% ethanol (1.88kg) were charged to a 10 liter flask equipped with a stirrer. The stirrer was started and the resin (1.5kg) was slowly added followed by Praziquantel (175g). When the Praziquantel was fully dispersed, the stirring was adjusted to maintain good suspension of both the resin and the Praziquantel. Stirring was overnight. The agitator was then stopped and the supernatent removed by filtration. 3.5kg of water was then added to the resin and the slurry stirred for 5 minutes. The supematent was then removed by filtration. The washing step was then repeated two more times. The resinate was dried in a vacuum oven at 60-70°C to a moisture content of <5%w/w. The dried resin contained 9.9% w/w praziquantel based on mass balance calculation.

### EXAMPLE 2

Release of Praziquantel from a Resinate.- In this example, the resinate used was prepared in a manner similar to Example 1 except that it was not dried for this test. It contained 9.3%w/w praziquantel (dry basis). 273.3mg of the resinate was added to 10ml of a solution of the following composition:
5.0% NaHCO3
2.35% NaCl
0.75% KCl
plus sufficient aq HCL to give a pH of 7.0

The mixture was shaken overnight. Observation of the mixture showed that a white precipitate was present, which was identified as praziquantel. Analysis of the supernatent for praziquantel showed that it's concentration was approximately at it's saturation limit. This example demonstrates that the praziquantel was released from the resin under conditions of approximately neutral pH where the resin was ionized.

### EXAMPLE 3

Praziquantel loaded onto an anion exchange resin. - In this example the resin used was a weakly basic anion exchange resin in its free base form. The resin had an exchange capacity of approximately 10meg/g. 100mg of praziquantel was added to 15g of 95% ethanol and shaken until it dissolved. 45g of water was then added followed by 3.1g of the resin (fully hydrated). The mixture was shaken overnight at room temperature and then filtered. The resinate contained 5.4 % praziquantel (dry basis).

### EXAMPLE 4

Epsiprantel loaded onto a cation exchange resin powder. In this example the resin used is the same as that used in Example 1 except that it is ground to a fine powder, having particles in the range 20-150 microns. A solution is prepared containing 20mg of epsiprantel in a mixture of 1.5g of 95 % ethanol and 4.5g of water. To this is added 1g of the cation exchange resin powder. This mixture is then shaken for approximately 24 hours. A sample of supematent is removed, filtered and analyzed for epsiprantel. The concentration of epsiprantel is between 800 and 1000mg/l. This concentration indicates that approximately 65 - 75% of the epsiprantel is loaded onto the resin.

### EXAMPLE 5

Treatment of tapeworm in cats. Six cats suffering from infestation with the common tapeworm *(Dipyllidium caninum)* are treated with 300mg of the resinate from Example 1, equivalent to approximately 30mg of praziquantel. The resinate is administered by addition to approximately 2 ounces of commercial, canned, cat food. All six cats consume the treated food within 5 minutes. One week later the cats are examined by a veterinarian for tapeworm infestation. All six are found to be free of infestation.

It is appreciated that in a variant of the invention one can selectively release one or more non-ionizable anthlemintics loaded onto a cation or anion exchange resin in different organs of an infected mammal. By way of example, a pharmaceutical composition is provided that includes a first therapeutically effective amount of a non-ionizable anthlemintic drug or derivative thereof loaded onto an anion exchange resin. Because of conditions in the stomach and intestines of an infected animal, the anthelmintic loaded onto an anion exchange resin will release under neutral and acidic conditions because the resin is ionized. Under acidic conditions, a cation exchange resin loaded with an anthelmintic is unionized. Hence, the anthelmintic loaded onto a cation exchange resin would generally not be released in the stomach of an infected animal. In the intestines, where there is about a neutral pH, both the anthelmintic loaded onto an anion exchange resin and the anthelmintic loaded onto a cation exchange resin as described herein would be released. It is further appreciated that a first dosage of anthelmintic loaded onto a cation exchange resin and a second dosage of anthelmintic loaded onto an anion exchange resin in a composition is manipulated so that effective therapeutic amounts of each respective resin are released in a desired dosage in a respective organ.

While only a few, preferred embodiments of the invention have been described hereinabove, those of ordinary skill in the art will recognize that the embodiment may be modified and altered without departing from the central spirit and scope of the invention. Thus, the preferred embodiment described hereinabove is to be considered in all respects as illustrative and not restrictive, the scope of the invention being indicated by the appended claims, rather than by the foregoing description, and all changes which come within the meaning and range of equivalency of the claims are intended to be embraced herein.

## Claims

1. A pharmaceutical composition comprising a non-ionizable anthlemintic drug or derivative thereof loaded onto an anion exchange resin or a cation exchange resin.

2. The pharmaceutical composition of claim 1 in which said non-ionizable anthlemintic is praziquantel, a praziquantel derivative, epsiprantel, or an epsiprantel derivative.

3. The pharmaceutical composition of claim 2 in which said composition further comprises a therapeutically effective dosage to treat a mammal, said therapeutically effective dosage providing for an anthelmintic spectrum of activity against one or more of Dipyllidium caninum, Taenia pisiformis, Echinococcus multilocularis, E. granulosus, and Taenia taeniaeformis, Toxocara, Ancylostoma, Uncinaria, Toxascaris, and Trichuris.

4. The pharmaceutical composition of claim 3 in which said therapeutically effective dosage is in the range of 3 to 100 mg/kg.

5. The pharmaceutical composition of claim 1 in which said anthlemintic drug is selected from the group consisting of Droncit, a derivative of Droncit, a precursor of Droncit, Drontal, a precursor of Drontal, a derivative of Drontal, Drontal Plus, a derivative of Drontal Plus, a precursor of Drontal Plus, a formulation comprising Praziquantel and Pyrantel Pamoate, and a formulation comprising Praziquantel, Pyrantel Pamoate and/or Febantel.

6. A pharmaceutical composition comprising a basic anthlemintic drug loaded onto an anion exchange resin.

7. A pharmaceutical composition comprising an acidic anthlemintic drug loaded onto a cation exchange resin.

8. A process for manufacturing a pharmaceutical composition comprising, loading onto a non-ionized form of a resin a non-ionized anthlemintic drug or derivative thereof.

9. A process for manufacturing a pharmaceutical composition comprising, loading onto an anion exchange resin in a non-ionized form a basic form of an anthlemintic drug or derivative thereof in a non-ionized from.

10. A process for manufacturing a pharmaceutical composition, comprising, loading onto a cation exchange resin in a non-ionized form an acidic form of an anthlemintic drug or derivative thereof in a non-ionized from.

11. A pharmaceutical composition comprising a first therapeutically effective amount of a non-ionizable anthlemintic drug or derivative thereof loaded onto an anion exchange resin, and a second therapeutically effective amount of said non-ionizable drug or derivative thereof loaded onto a cation exchange resin, whereby selective release of said drug is provided *in vivo*.
